# EUROPEAN PATENT APPLICATION

(11) **EP 1 293 227 A1**
(43) Date of publication of application: **19.03.2003**
(21) Application number: 02018595.5
(22) Date of filing: 19.08.2002
(51) Int. Cl.: A61M 16/06

(54) **Headgear for nasal masks**

(30) Priority: 20.08.2001 NZ 51365801
(71) Applicant: Fisher & Paykel Healthcare Limited, East Tamaki, Auckland (NZ)
(72) Inventor: Smith, Nicholas Charles Alan, Onehunga, Auckland (NZ); McAuley, Alastair Edwin, Remuera, Auckland (NZ); Nightingale, Chris Earl, Ellerslie, Auckland (NZ); Milivojevic, Ivan, Takapuna, Auckland (NZ); Gradon, Lewis George, Howick, Auckland (NZ)
(74) Representative: Brown, John David

(57) **Abstract**

A nasal mask (501) is disclosed which is more comfortable for the user to wear and reduces side leakage as compared to masks of the prior art. In a first form headgear (502) for securing a mask to the user's face is disclosed where a section of at least one strap securing a mask to a user's face is substantially more elastic then the remainder of the strap. Headgear of this type allows the user to tilt the head backwards without lateral movement of the mask. In a further aspect, headgear tensioners (506,507) are disclosed which provides a preset fitting of the mask and ensures that a user cannot over tighten the straps. The tensioners are recalling tension devices attached to the straps and/or mask on either side of the mask. In another form the headgear tensioner is a strap attachment on either side of the mask that allows the straps to move back through the attachment to a predetermined position that provides an appropriate tension between the back of the user's head and mask. This limits the amount of leakage from the mask when the straps tighten or loosen as the user moves his/her head.

## Description

### BACKGROUND OF THE INVENTION

### Field of the Invention

This invention relates to headgear and nasal masks particularly though not solely for use in providing Continuous Positive Airway Pressure (CPAP) therapy to patients suffering from obstructive sleep apnoea (OSA).

### Summary of the Prior Art

In the art of respiration devices, there are well known a variety of respiratory masks which cover the nose and/or mouth of a human user in order to provide a continuous seal around the nasal and/or oral areas of the face such that gas may be provided at positive pressure within the mask for consumption by the user. The uses for such masks range from high altitude breathing (ie. aviation applications) to mining and fire fighting applications, to various medical diagnostic and therapeutic applications.

One requisite of such respiratory masks has been that they provide an effective seal against the user's face to prevent leakage of the gas being supplied. Commonly, in prior mask configurations, a good mask-to-face seal has been attained in many instances only with considerable discomfort for the user. This problem is most crucial in those applications, especially medical applications, which require the user to wear such a mask continuously for hours or perhaps even days. In such situations, the user will not tolerate the mask for long durations and optimum therapeutic or diagnostic objectives thus will not be achieved, or will be achieved with great difficulty and considerable user discomfort.

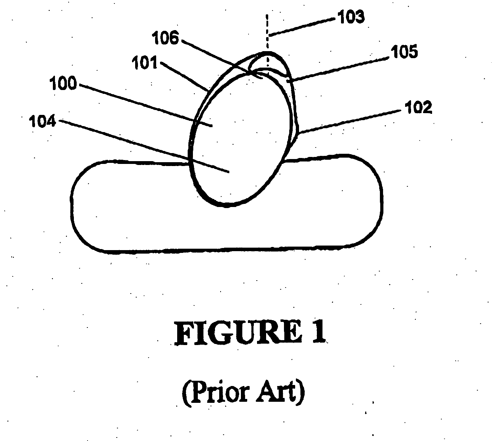

In common with prior art designs is an inability to seal effectively when the user's face becomes distorted. For example, as shown in the prior art mask of Figure 1 when the user 100 is sleeping on his or her side, one side 101 of the headgear tends to be pulled tight while the other side 102 tends to be loose. This causes the axis of the mask 103 to be twisted with respect to the axis of the head 104, due to the net torque from the headgear, resulting in leakage 105 on one side. The user 100 sleeping on his or her side may also distort the facial contours around the nasal area 106 and may lead to further leakage. More importantly, if a user tilts their head either forward or backwards, the distance between the mask attachment and the back of the head changes, thus causing loosening of the mask causing leakage of gases from the mask or tightening of the mask on the users face causing discomfort and in some cases pressure sores.

### SUMMARY OF THE INVENTION

It is an object of the present invention to provide a nasal mask which goes some way to overcoming the abovementioned disadvantages in the prior art or which will at least provide the industry with a useful choice.

In a first aspect the present invention may broadly be said to consist in headgear for securing a mask to a user's face, comprising or including;
at least one strap having two ends each adapted to in use be attached to a respective side of said mask and around the back of the user's head,
wherein a section of said at least one strap is substantially more elastic than the remainder of said at least one strap.

In a second aspect the present invention may broadly be said to consist in a nasal mask securable to a user's face, comprising or including:
restraining means attached to or around the head of said user,
a body portion having an inlet receiving said supply of gases and an open section,
sealing means attached to said body portion substantially contoured to the facial contours of said user, and
a receiving means attached to said body which in use engages with said restraining means,
said restraining means comprising or including:
   a least one strap having two ends each capable of being releasably attached to said receiving means, said at least one strap extending, in use, from one side of said mask, around the back of the user's head to the other side of said mask,
wherein a section of said at least one strap Is substantially more elastic than the remainder of said at least one strap.

In a third aspect the present invention may broadly be said to consist in headgear for securing a mask to a user's face comprising or including:
at least one strap having two ends, where at least one end adapted to, in use, be fixed to a side of said mask,
a tensioning device, adapted to attach to one of the end of at least one strap not attached to said mask and said mask, or integrated as part of said at least one strap, which provides a tension between said strap and said mask, thereby restraining said mask on the face of said user.

In a fourth aspect the present invention may broadly be said to consist in headgear for securing a mask to a user's face comprising or including;
at least one strap having two ends, where at least one end is adapted to, in use, be fixed to a side of said mask, around the back of the user's head to the other side of said mask, each of said side attachment means comprising or including:
   a flame attached to said mask, said frame including an opening, and
   a tongue within said opening, said tongue having a first end connected with said frame and a second end freely moveable, said second end including a gripping surface and said opening including a sliding surface,
   wherein a gap between said gripping surface and said sliding surface is of a distance such that when said strap is in use passed through said gap said second end is moved out of alignment with said frame allowing the length of said strap to be pulled through said gap,
   wherein, in use, when said strap is pulled through said gap to an over tensioned condition, said strap will slip through said gap until a balance tension is reached, said balance tension appropriate to substantially located said mask on the face of said user.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

### BRIEF DESCRIPTION OF THE DRAWINGS

One preferred form of the present invention will now be described with reference to the accompanying drawings in which;
**Figure 1** is a plan view of a prior art mask and headgear illustrating side leak,
**Figure 2** is block diagram of a humidified continuous positive airway pressure system as might be used in conjunction with the present invention,
**Figure 3** is a view of a prior art mask from the front in a configuration as if worn by the user, but not showing the users head,
**Figure 4** is a view of the mask and headgear of the present invention, as shown in an extended unused form,
**Figure 5** is a view of an alternative form of the mask and headgear of the present invention shown in a configuration as if worn by the user, but not showing the users head, where the headgear upper straps are provided with a recoil device,
**Figure 6** is a cross-sectional view of the recoil device through AA in Figure 5,
**Figure 7** is a plan view of the recoil device showing the interior details of the device,
**Figure 8** is a view of a further alternative form of the mask and headgear of the present invention shown in a configuration as if worn by the user, but not showing the users head, where the headgear upper straps are provided with an auto-tensioning device,
**Figure 9** is a plan view of one auto-tensioning device of the further alternative form of the present invention, and
**Figure 10** is a side view of the auto-tensioning device, showing the tongue flexing downwards under the force of the strap.

### DETAILED DESCRIPTION OF THE PREFERRED EMBODIMENTS

The present invention provides improvements in the field of nasal masks for use in positive pressure ventilation. In particular a nasal mask is described which is more comfortable for the user to wear and reduces the side leakage as compared with masks of the prior art. It will be appreciated that the nasal mask as described in the preferred embodiment of the present invention can be used in respiratory care generally or with a ventilator but will now be described below with reference to use in a humidified positive pressure ventilation system.

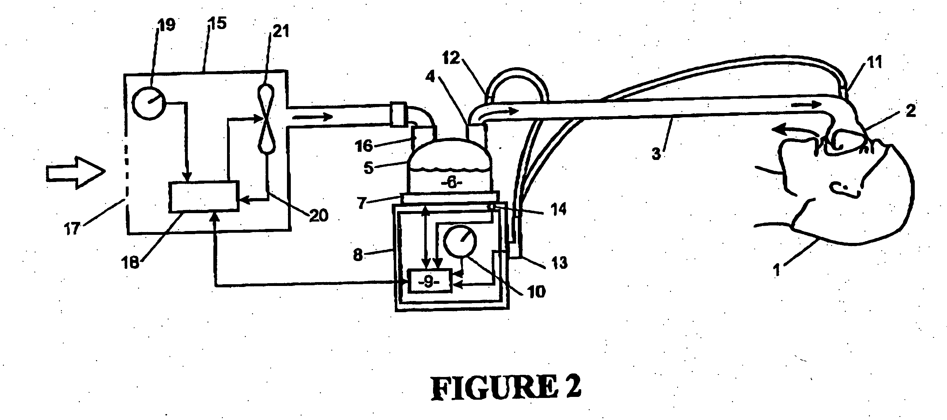

With reference to Figure 2 a humidified positive pressure ventilation system is shown in which a user 1 is receiving humidified and pressurised gases through a nasal mask 2 connected to a humidified gases transportation pathway or inspiratory conduit 3. It should be understood that delivery systems could also be VPAP (Variable Positive Airway Pressure) and BiPAP (Bi-level Positive Airway Pressure) or numerous other forms of respiratory therapy. Inspiratory conduit 3 is connected to the outlet 4 of a humidification chamber 5 which contains a volume of water 6. Inspiratory conduit 3 may contain heating means or heater wires (not shown) which heat the walls of the conduit to reduce condensation of humidified gases within the conduit. Humidification chamber 6 is preferably formed from a plastics material and may have a highly heat conductive base (for example an aluminium base) which is in direct contact with a heater plate 7 of humidifier 8. Humidifier 8 is provided with control means or electronic controller 9 which may comprise a microprocessor based controller executing computer software commands stored in associated memory.

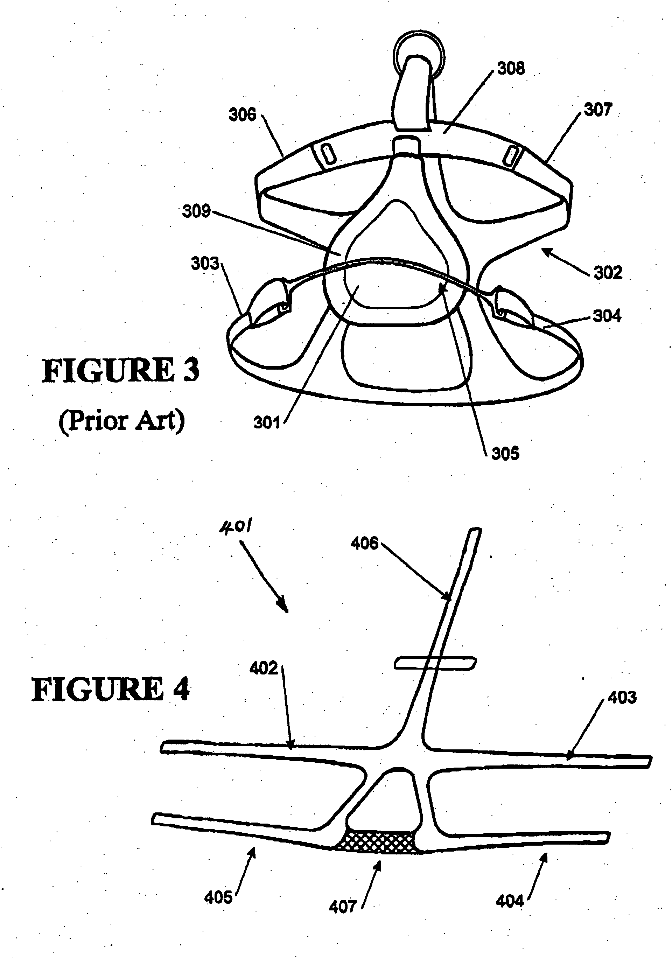

Controller 9 receives input from sources such as user input means or dial 10 through which a user of the device may, for example, set a predetermined required value (preset value) of humidity or temperature of the gases supplied to user 1. The controller may also receive input from other sources, for example temperature and/or flow velocity sensors 11 and 12 through connector 13 and heater plate temperature sensor 14. In response to the user set humidity or temperature value input via dial 10 and the other inputs, controller 9 determines when (or to what level) to energise heater plate 7 to heat the water 6 within humidification chamber 5. As the volume of water 6 within humidification chamber 5 is heated, water vapour begins to fill the volume of the chamber above the water's surface and is passed out of the humidification chamber 5 outlet 4 with the flow of gases (for example air) provided from a gases supply means or blower 15 which enters the chamber through inlet 16. Exhaled gases from the user's mouth are passed directly to ambient surroundings in Figure **3.**

Blower 15 is provided with variable pressure regulating means or variable speed fan 21 which draws air or other gases through blower inlet 17. The speed of variable speed fan 21 is controlled by electronic controller 18 (or alternatively the function of controller 18 could carried out by controller 9) in response to inputs from controller 9 and a user set predetermined required value (preset value) of pressure or fan speed via dial 19.

### Nasal Mask

Referring to Figures 2 and 3, the nasal mask (generally indicated as 301) as used with headgear of the present invention includes a hollow body 301 with an inlet connected to the inspiratory conduit The mask 301 is positioned around the nose of the user 1 with the headgear 302 secured around the back of the head of the user. The restraining force from the headgear 302 on the hollow body 301 and the forehead rest 308 ensures enough compressive force on the mask cushion 309, to provide an effective seal against the user's face.

The hollow body of the mask 301 is constructed of a relatively inflexible material for example, polycarbonate plastic. Such a material would provide the requisite rigidity as well as being transparent and a relatively good insulator. The expiratory gases can be expelled through a valve (not shown) in the mask, a further expiratory conduit (not shown), or any other such method as is known in the art.

### Hybrid Headgear

Existing respiratory masks are usually made from a standard neoprene or the like material and are provided in a modified H-type configuration as shown in Figure 3. A respiratory mask 301 is attached to a user's head using the headgear 302. The headgear comprises lower side straps 303, 304 that each attach to respective ends of a sliding strap 305. The sliding strap 305 is attached and slideable in and across the mask 301 in guides (not shown) moulded into the mask 301 that constrains the sliding strap 305, but allows it to slide over the mask, meaning the headgear 302 can move laterally, independent of the mask 301.

The advantage in having a sliding strap is that as the face is contorted during various sleeping positions the headgear is able to move with the changes in position while the mask is left in the correct position on the nose of the user and an effective seal is maintained.

The headgear 302 also comprises upper straps 306, 307 that attach to the respective sides of the T-shaped forehead rest 308. The upper and lower straps meet behind the users head and form a triangular shaped partial skull cap. In this form, if the user tilts their head upwards the headgear tension increases between the back of the user's head and the mask and the mask 301 moves towards the user's face and mouth putting more pressure on the user's upper lip making the mask and headgear tight and uncomfortable for the user. Also, the user tilts their head forward (downwards) there is a reduction of tension in the straps and they slacken causing the mask to loosen and gas to leak from the mask.

To overcome this, in a first form as illustrated in Figure 4, the present invention provides headgear 401 that is used with a respiratory mask as already described. The headgear 401 has upper 402, 403 and lower 404, 405 side straps and a top strap 406. In order to allow the user to tilt their head backwards without increased pressure on the users face and upper lip due to increased tension in the lower side straps 404, 405, the lower strap is provided with a flexible section 407 made from a material that is more flexible that the material making up the remainder of the strap. In use, the headgear 401, is placed over and around a user's head and the upper straps 402, 403 are attached by appropriate means, for example, a buckle or using VELCRO™, to the forehead rest 308 (see Figure 3). The lower straps are attached, again by any appropriate means, to the slideable strap 305 (see Figure 3). The flexible section 407, due to it's elasticity, extends when the user tilts their head forward preventing the mask from being pulled uncomfortably against the user's face. Consequently, when the user tilts their head upwards the flexible section 407 retracts causing the seal between the mask and the user's face to remain, maintaining the mask against the user's face.

### Recoiling Headgear Tensioner

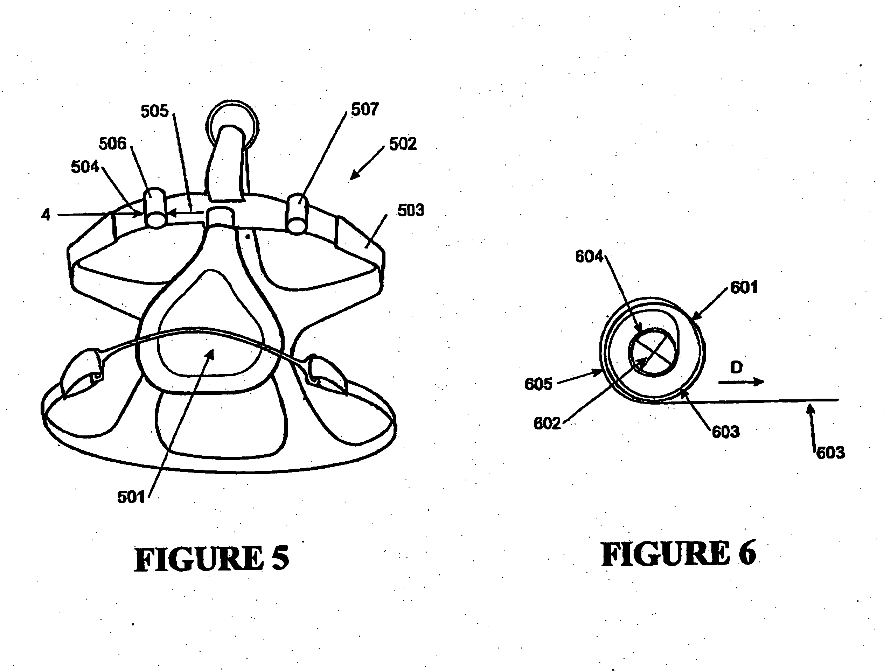

Current problems with existing headgear used with nasal masks is that a user when putting on the nasal mask for use tends to over tighten the upper straps of the headgear. Also, traditionally headgear of this type is difficult and time consuming to fit into a comfortable position, but yet tight enough to provide the appropriate sealing of the mask upon the user's face. Figure 5 shows an alternative form of headgear that may be provided with a nasal mask that utilises a constant tension system. This headgear provides a pre-set fitting of the mask and ensures that the user cannot over tighten the straps. Here, the headgear 502 attaches to a nasal mask 501 much in the same way as that form as shown in Figure 4, but each of the upper straps 503, 504 are attached to a forehead rest 505 by way of recoiling tension devices 506, 507. It must be noted that in the following embodiment recoiling tension devices are provided on the upper straps only. It is possible for the headgear and mask to be provided with recoiling tension devices on the lower straps or in fact on all straps connecting the headgear to the mask, therefore the description following must not be interpreted as being limited to two recoiling tension devices on the upper straps. One such recoiling tension devices 601 is shown in cross-section in Figure 6. This device 601 comprises a spool 602 that has attached to it the strap 603. The spool 602 is biased by a spiral, clock-type spring 604, to continually urge the spool to wind the strap around the spool, but allows the strap to be pulled from the retraction device 601 when a force, indicated by the arrow D, is placed upon the strap by the user. The spring is wound with a preselected tension that subsequently winds the strap around the spool to a certain position that provides an appropriate tension between the back of the user's head and mask.

As shown in Figure 6, the strap 603 may be wound around and over the spring 604 within the one compartment or drum 605. Alternatively, as described below the spring may be in a separate side compartment than that of the strap.

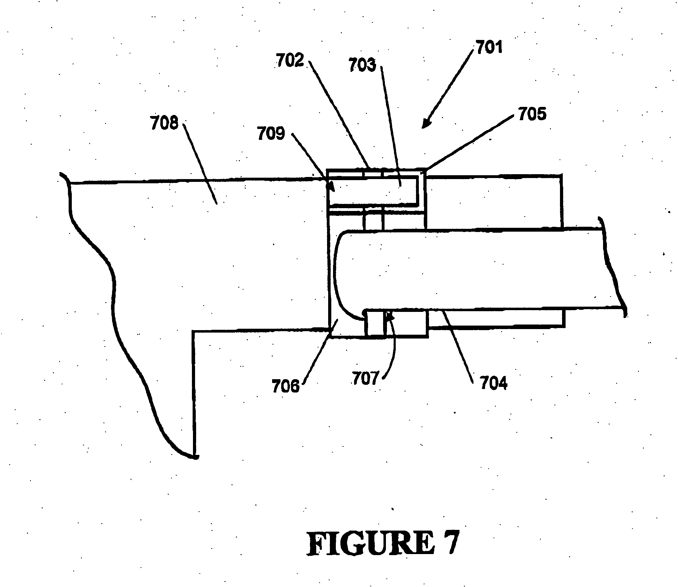

Figure 7 shows a further view of the interior of such a recoiling tension device 701, the housing containing the spool 702, spring 703 and wound strap 704 is split into two compartments 705, 706. The first compartment 705 houses the spring 703 and part of the spool 702. The remainder of the spool 702 extends through into the second compartment 706 having an elongated hole in it's side (not shown) in which the strap 704 extends from. The strap end 707 is fixed to the spool 702 within the second compartment to enable the winding of the strap around the spool. Furthermore, the strap must be made of a material that is sufficiently thin enough to be wound around the spool, yet strong enough not to break under strain. Therefore, at least the length of the strap wound around the spool will be made from such an appropriate material, whereas, the rest of the strap extending around the user's head may be made of a stronger and thicker material. As shown in Figures 5 and 7 the recoiling tension device (501 or 701) is located on the forehead rest (505 or 708) and is fixed by appropriate attachment means, for example, a rigid attachment or by a clipping mechanism.

Additionally, it is anticipated that each recoiling tension device will be adjustable, so as to ensure an appropriate tension for different head sizes of potential users. It is envisaged that the spring tension would be adjusted by fixing one end 709 of the spring 703 to the interior of compartment 705 and the compartment 705 being rotatably mounted to the fixed compartment containing the wound strap, so that in effect compartment 705 is a rotatable drum about the fixed spool 702. The rotation of the drum 705 about the spool effectively provides for the tightening of the spring in one direction and loosening the spring in the opposite direction, therefore allowing for the tension In the spring to be adjustable, and thus for the tightening or loosening of the straps around the user's head.

### Flexible Headgear Tensioner

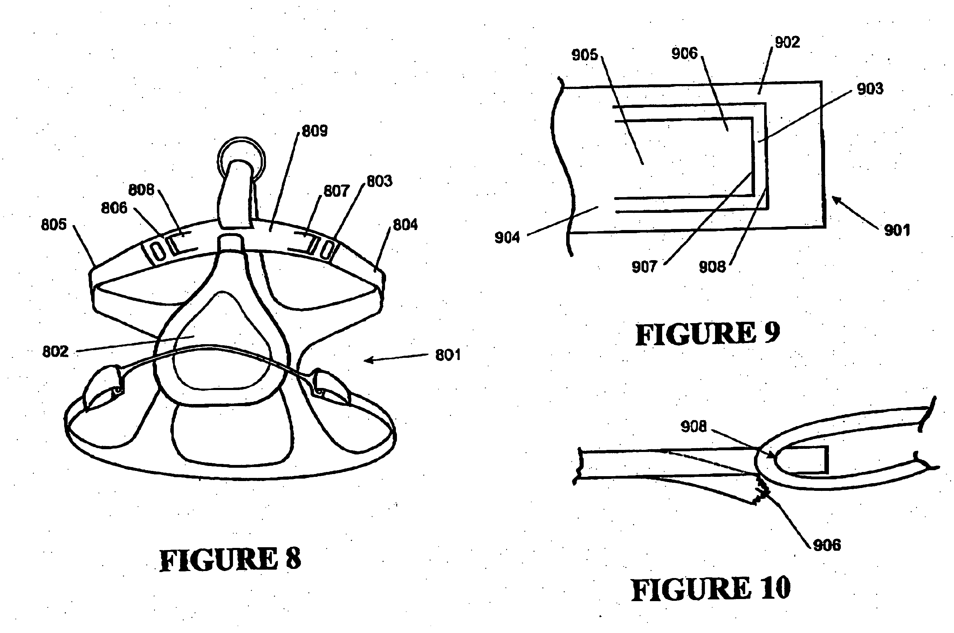

A further alternative form of the present invention provides a strap attachment to the mask that can be used to automatically adjust the tension in the mask headgear straps. Figure 8 shows a mask 801 utilising such headgear, where during use, the user would place the mask 802 over their nasal and airway passages, then adjust the headgear 803 and tighten the upper straps 804, 805 about their head. If the user over tightened the straps the strap attachment 806, 807 of the present Invention will allow each end of the strap to move back through the attachment to a predetermined position that provides an appropriate tension between the back of the user's head and mask. Again, in the following embodiment strap attachments are provided on the upper straps only. The present invention can extend to headgear and mask that includes strap attachments on the lower straps or in fact on all straps of the headgear.

Figure 9 shows a plan view of the strap attachment 901 of the further alternative form of the present invention This attachment is located on both arms of the forehead rest 808, 809 and may either be moulded into the end of the arm or the attachment 901 may be provided with a clipping mechanism that clips an appropriate complementary mechanism provided at either end of the arms of the forehead rest 808, 809. The attachment 901 is a frame 902 defining an opening 903. Within the opening 903, and in moulded connection with one end 904 of the frame 902 is a flexible tongue 905. The other end of the tongue 906 is able to freely move up and down within the opening 903 and is provided with an serrated or ridged edge 907 that provides a gripping force on the strap as it is threaded through the gap provided between the tongue end 906 and the part of the frame surface 908. The interior of the frame surface 908 is rounded to allow the strap to slide over the frame surface and through the gap.

In use, when a user attaches the mask to their face, threads the straps 804, 805 (see Figure 8) through each opening 903, and pulls the straps into an over tight position by pulling more of the length of the strap through the gap, as shown in Figure 10 the free end of each tongue 906 will flex downwards under the force of the strap. When the user releases the strap, the length of the strap will slip back through the opening 903 as the flexible tongue moves back into it's original position. The backward slipping of the strap will be stopped at a certain point by the gripping surface 907 of the tongue end. This point is determined by a preselected tension as set by the flexibility, and thus the material, of the tongue. This point is that which provides an appropriate tension between the back of the user's head and mask. The preselected tension that is appropriate to fix the mask to the user's face and to limit the amount of leakage from the mask when the straps tighten or loosen as the user moves his or her head is between 1 and 10 Newtons.

In the present specification "comprises" means "includes or consists of" and "comprising" means "including or consisting of'.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. Headgear for securing a mask to a user's face, comprising or including:
at least one strap having two ends each adapted when in use to be attached to a respective side of said mask and around the back of the user's head,
wherein a section of said at least one strap is substantially more elastic than the remainder of said at least one strap, said section in use assisting to maintain an appropriate tension to locate said mask on the face of said user.

2. Headgear for securing a mask to a user's face according to claim 1 wherein said mask includes two upper and two lower attachment means where said ends of said at least one strap are respectively releasably attached to said two lower attachment means,
said headgear further including a second strap having two ends each capable of being attached to a respective of said two upper attachment means,
wherein, in use, said second strap extends from one side of said mask, around the back of the user's head to the other side of said mask.

3. Headgear for securing a mask to a user's face according to any one of claims 1 or 2 wherein said at least one strap and said second strap are attached to one another by at least one connecting means.

4. Headgear for securing a mask to a user's face according to claim 3 wherein said connecting means is at least one substantially longitudinal strap connecting the latitudinally orientated said at least one strap and said second strap.

5. A nasal mask securable to a user's face, comprising or including:
restraining means attached to or around the head of said user,
a body portion having an inlet receiving said supply of gases and an open section,
sealing means attached to said body portion substantially contoured to the facial contours of said user, and
a receiving means attached to said body which in use engages with said restraining means,
said restraining means comprising or including:
a least one strap having two ends each capable of being releasably attached to said receiving means, said at least one strap extending, in use, from one side of said mask, around the back of the user's head to the other side of said mask,
wherein a section of said at least one strap is substantially more elastic than the remainder of said at least one strap, said section in use assists to maintain an appropriate tension to locate said mask on the face of said user.

6. A nasal mask securable to a user's face according to claim 5 wherein said restraining means includes two upper and two lower attachment means where said ends of said at least one strap are respectively releasably attached to said two lower attachment means,
said headgear further including a second strap having two ends each capable of being releasably attached to a respective of said two upper attachment means,
wherein, in use, said second strap extends from one side of said mask, around the back of the user's bead to the other side of said mask.
